# EUROPEAN PATENT APPLICATION

(11) **EP 4 623 921 A1**
(43) Date of publication of application: **01.10.2025**
(21) Application number: 23894798.0
(22) Date of filing: 06.10.2023
(51) Int. Cl.: A61K 35/545, A61K 35/28, A61P 9/00, A61P 25/00, C12N 5/0775

(54) **VMSC SECRETOME AND USE THEREOF**

(30) Priority: 25.11.2022 KR 20220160209; 28.07.2023 KR 20230099113
(71) Applicant: Elphis Cell Therapeutics, Seoul 02455 (KR)
(72) Inventor: SON, Young Sook, Seoul 06305 (KR); PARK, Ki Sook, Seoul 06374 (KR); JANG, Hyun Hee, Seoul 04780 (KR); PARK, Ga Bee, Asan-si Chungcheongnam-do 31533 (KR)
(74) Representative: Isarpatent
(86) International application number: PCT/KR2023/015368
(87) International publication number: WO 2024/111868

(57) **Abstract**

The present disclosure relates to a newly identified vMSC secretome and use thereof. Unlike conventional MSCs, vMSCs of the present disclosure, obtained through culturing under specific culture conditions, are novel MSCs in which a CD141 cell surface antigen is expressed and the compositions of secretomes secreted by the vMSCs are different from those of conventional MSCs, and which remarkably increase vascular sprouting of other stem cells, and thus present disclosure can be used for promoting vascularization of stem cells and for preventing or treating vascular diseases or vascular dysfunction.

## Description

### [Technical Field]

The present disclosure relates to a newly identified vMSC secretome and use thereof.

### [Background Art]

New blood vessels are generated by angiogenesis, arteriogenesis and vasculogenesis. The angiogenesis is related to the proliferation and migration of endothelial cells that grow from already existing mature endothelial cells, the arteriogenesis is a process of remodeling already existing arteriolar connections into collateral vessels, and the vasculogenesis proceeds through a process in which endothelial progenitor cells differentiate into mature endothelial cells. Therefore, the endothelial progenitor cells circulating from the bone marrow migrate to a vascular damage area, and are involved in direct insertion into newly formed blood vessels or new blood vessel formation through various vascularization and secretion of trophic factors. Therefore, the endothelial progenitor cells are attracting attention as potential targets in the field of regenerative medicine and for therapeutic purposes through revascularization. Accordingly, studies have been conducted to increase the function of the endothelial progenitor cells, and for example, studies on the production of recombinant EPCs obtained by modifying *ex vivo* genes have been conducted, and a technique for increasing the proangiogenic capacity of EPCs using a vascular endothelial growth factor (VEGF) or hypoxia inducible factor-1α has been studied. They are largely divided into two types of cells that are currently used as cell therapy in ischemic vascular diseases. There are endothelial progenitor cells (EPCs) that play a role in vascular regeneration and mesenchymal stem cells that secrete large amounts of growth factors that contribute to vasculogenesis and have a paracrine effect that indirectly helps vasculogenesis. However, existing EPCs are a cell group in which *in vitro* cell expansion is very difficult, and it is also difficult to maintain function during cell expansion, and there is a high risk of problems such as immune rejection in the case of allogeneic and xenogeneic cells, and have a limitation that it is difficult to differentiate and function into blood vessels with complex structures. In addition, conventional therapy products developed based on mesenchymal stem cells targeting ischemic diseases are lost without engraftment and thus do not exhibit a clear therapeutic effect in clinical trials by depending on the paracrine effect rather than angiogenesis-based treatment.

Cell therapy products, including stem cell therapy products, are defined as medicines used for treatment, diagnosis, and prevention through a series of actions, such as proliferating or selecting living autologous, allogeneic, or xenogeneic cells *in vitro* or changing the biological characteristics of cells through other methods to restore the functions of cells and tissues. Cell therapy transplantation using cell therapy products is used for treatment by separating and preparing each cell material composed of a plurality of cells capable of replacing various tissues that constitute the body and have been expressed as tissues similar to the normal or healthy state in the body, performing a series of actions for changing the biological characteristics of cells through a culture process in an in vitro environment, and then injecting the cell material into an individual to receive an implant or creating human tissues with these cells. The stem cell therapy product refers to a cell therapy product that specifically uses stem cells, and currently, research has been actively conducted in representative application fields where recovery and regeneration of lost cells are essential, but not well achieved, such as neurological diseases, heart diseases, lung diseases, liver diseases, and cancer. Stem cells have great potential in cell therapy because the stem cells have the multiple differentiating potential to induce differentiation into the cells needed in damaged tissues. However, at the current level of development, the survival rate after transplantation into the body is not high, so that actually, it is difficult to find examples of widespread and stable success in clinical application.

### [Disclosure]

### [Technical Problem]

An object of the present disclosure is to provide a novel secretome secreted by stem cells.

Another object of the present disclosure is to provide a pharmaceutical composition for vascular regeneration or vasculogenesis.

Yet another object of the present disclosure is to provide stem cells with enhanced proangiogenic capacity.

Still another object of the present disclosure is to provide a cell therapy product composition for vascular regeneration or angiogenesis.

Still another object of the present disclosure is to provide a pharmaceutical composition for preventing or treating vascular diseases or vascular dysfunction.

Still another object of the present disclosure is to provide a pharmaceutical composition for preventing or treating brain nervous system diseases.

Still another object of the present disclosure is to provide a method for producing the novel stem cell-derived secretome.

Still another object of the present disclosure is to provide a use of secretomes secreted by stem cells expressing a CD141 cell surface antigen.

Still another object of the present disclosure is to provide a use of stem cells treated with secretomes secreted by stem cells expressing a CD141 cell surface antigen or a culture solution of the stem cells expressing the CD141 cell surface antigen.

Still another object of the present disclosure is to provide a method for treating vascular diseases or vascular dysfunction.

Still another object of the present disclosure is to provide a method for preventing or treating brain nervous system diseases.

### [Technical Solution]

In order to achieve the objects, an aspect of the present disclosure provides a novel secretome secreted by stem cells.

Another aspect of the present disclosure provides a pharmaceutical composition for vascular regeneration or vasculogenesis, including a culture solution or secretomes of novel stem cells.

Yet another aspect of the present disclosure provides stem cells treated with secretomes secreted by the novel stem cells.

Still another aspect of the present disclosure provides a cell therapy product composition for vascular regeneration or vasculogenesis including the stem cells.

Still another aspect of the present disclosure provides a pharmaceutical composition for preventing or treating vascular disease or vascular dysfunction, including the secretomes or stem cells treated with the secretomes.

Still another aspect of the present disclosure provides a pharmaceutical composition for preventing or treating brain nervous system diseases.

Still another aspect of the present disclosure provides a method for producing the novel stem cell-derived secretomes.

Still another aspect of the present disclosure provides a use of secretomes secreted by stem cells expressing a CD141 cell surface antigen, for use in vascular regeneration or vasculogenesis.

Still another aspect of the present disclosure provides a use for vascular regeneration or vasculogenesis of stem cells treated with secretomes secreted by stem cells expressing a CD141 cell surface antigen or a culture solution of the stem cells expressing the CD141 cell surface antigen.

Still another aspect of the present disclosure provides a method for treating vascular diseases or vascular dysfunction.

Still another aspect of the present disclosure provides a method for preventing or treating brain nervous system diseases.

### [Advantageous Effects]

According to the present disclosure, unlike conventional mesenchymal stem cells or mesenchymal stromal cells (MSCs), vasculogenic multipotent stem cells (vMSCs), obtained through culturing under specific culture conditions, are novel MSCs in which a CD141 cell surface antigen is expressed while sharing various characteristics with conventional MSCs, the compositions of secretomes secreted by the vMSCs are different from those of conventional MSCs, and which remarkably increase vascular sprouting of other stem cells, and thus present disclosure can be used for promoting vascularization of stem cells and for preventing or treating vascular diseases or vascular dysfunction.

### [Description of Drawings]

FIG. 1 is a diagram showing colony shapes of CD141⁺vMSCs formed in various extracellular matrices.
FIG. 2 is a diagram confirming the cell characteristics according to expression of CD141⁺vMSC markers.
FIG. 3 is a diagram showing results of discovering novel markers specific to CD141⁺vMSCs that are distinguished from BM-MSCs through marker screening.
FIG. 4 is a diagram showing results of analyzing cytokines involved in vasculogenesis in secretome secreted by vMSCs using the Human Angiogenesis Array:
   FIG. 4A: Results of analyzing only a culture solution (EGM-2-FBS+2%hPL) of CD141⁺vMSCs (control group);
   FIG. 4B: Results of analyzing secretomes obtained by culturing CD141⁺vMSCs in a culture solution;
   FIG. 4C: Difference values (fold changes) of factors significantly increased in CD141⁺vMSC secretomes compared to the control group; and
   FIG. 4D: Quantitative measurement results of Mean Pixel Density of the blot.
FIG. 5 is a diagram showing results of analyzing cytokines involved in vasculogenesis in secretome secreted by BM-MSCs using the Human Angiogenesis Array:
   FIG. 5A: Results of analyzing only a culture solution (StemMACS^{™}MSC expansion media) of BM-MSCs (control group);
   FIG. 5B: Results of analyzing secretomes obtained by culturing BM-MSCs in a culture solution;
   FIG. 5C: Difference values of factors significantly increased in BM-MSC secretomes compared to a control group; and
   FIG. 5D: Quantitative measurement results of Mean Pixel Density of the blot.
FIG. 6 is a diagram showing results of ELISA analysis of HGF levels in secretomes secreted by vMSCs:
   FIG. 6A: HGF concentrations in CD141+vMSC secretomes at passage 3 from various donors;
   FIG. 6B: HGF concentration secreted per cell obtained by dividing HGF concentrations in CD141⁺vMSC secretomes at passage 3 from various donors by cell number; and
   FIG. 6C: HGF levels in secretomes obtained by culturing CD141⁺vMSCs and BM-MSCs at passage 3 in growth factor-deficient culture conditions (MEMa+0.2%hPL).
FIG. 7 is a diagram showing results of ELISA analysis of VEGF levels in secretomes secreted by BM-MSCs:
   FIG. 7A: VEGF concentrations in BM-MSC secretomes at passage 3 from various donors;
   FIG. 7B: VEGF concentration secreted per cell obtained by dividing VEGF concentration in BM-MSC secretomes at passage 3 from various donors by the cell number; and
   FIG. 7C: VEGF levels in secretomes obtained by culturing CD141⁺vMSCs and BM-MSCs at passage 3 in growth factor-deficient culture conditions (MEMa+0.2%hPL).
FIG. 8 is a schematic diagram of a method for producing spheroids using HUVEC cells and non-contact co-culture with vMSCs using the method.
FIG. 9 is a diagram confirming sprouting ability of HUVEC spheroids in response to HGF which is one of secretomes of vMSC.
FIG. 10 is a schematic diagram of a method for producing spheroids using vMSC cells and treatment with angiogenesis promoting factors or non-contact co-culture with vMSCs using the same.
FIG. 11 is a diagram showing sprouting ability of CD141⁺vMSCs spheroids for VEGF and bFGF.
FIG. 12 is a diagram showing sprouting ability of CD141⁺vMSCs spheroids for HGF.
FIG. 13 is a diagram showing vMSC sprouting promoting effects of vMSC spheroids and secretomes of vMSCs through non-contact co-culture with vMSCs and HGF among the secretomes.
FIG. 14 is a schematic diagram of a method for producing spheroids using vMSC cells and bFGF treatment or non-contact co-culture with BM-MSC using the method.
FIG. 15 is a diagram confirming mRNA expression levels of bFGF in vMSCs and BM-MSCs:
   MSCs: BM-MSC.
FIG. 16 is a diagram confirming the sprouting ability of vMSC spheroids by treatment with bFGF secreted in large quantities by BM-MSCs or by treatment with its inhibitor:
   PD173074: bFGF signaling pathway inhibitor.
FIG. 17 is a diagram showing vMSC sprouting promoting effects of vMSC spheroids and secretomes of BM-MSC through non-contact co-culture with BM-MSC and bFGF among the secretomes:
   MSCs: BM-MSC; and
   PD173074: bFGF signaling pathway inhibitor.

### [Best Mode of the Invention]

Hereinafter, embodiments of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used herein are terminologies used to properly express preferred exemplary embodiments of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to imply the inclusion of stated elements, not the exclusion of any other elements.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications disclosed as references in this specification are incorporated in the present disclosure.

Throughout this specification, '%' used to indicate the concentration of a specific material is solid/solid (w/w)%, solid/liquid (w/v)%, and liquid/liquid (v/v)%, unless otherwise stated.

In one aspect, the present disclosure relates to a culture solution of stem cells expressing a CD141 (thrombomodulin TM) cell surface antigen.

In one embodiment, the stem cells expressing the cell surface antigen CD141 may be vasculogenic multipotent stem cells (vMSCs).

In one embodiment, the stem cells expressing the cell surface antigen CD141 may be CD141⁺Vasculogenic Multipotent Stem Cells (CD141⁺vMSCs) deposited under the accession number KCLRF-BP-00524.

In one embodiment, the culture solution of the stem cells expressing the CD141 cell surface antigen may be a culture or a supernatant thereof obtained during or after culturing the cells in a medium. The culture solution of the stem cells expressing the CD141 cell surface antigen may be a concentrate of a culture, a filtrate thereof, a fraction thereof, or a supernatant thereof, a lyophilized product thereof, or a supercritical dried product thereof obtained during or after culturing the cells. The concentrate may be obtained by concentrating a culture of stem cells expressing the CD141 cell surface antigen or a supernatant thereof about 2-fold, about 3-fold, about 4-fold, about 5-fold, about 6-fold, about 7-fold, about 8-fold, about 9-fold, about 10-fold, or about 20-fold, and may be obtained by removing apoptosomes and cell debris using a filter having a diameter of 200 to 600 nm, and concentrating the culture about 2-fold, about 3-fold, about 4-fold, about 5-fold, about 6-fold, about 7-fold, about 8-fold, about 9-fold, about 10-fold, or about 20-fold using an ultracentrifuge.

In one embodiment, the filtrate may be obtained by filtering the culture solution to remove apoptosomes (about 1000 to 600 nm in size), which are microbodies associated with cell death.

In one embodiment, the culture solution of the stem cells expressing the CD141 cell surface antigen may include secretomes secreted outside the cells by producing bioactive molecules through intercellular interactions during the process of culturing the cells. The secretomes may include substances such as cell-derived proteins, cytokines, growth factors, exosomes, nucleic acids (DNA and RNA), and the like, and various bioactive molecules such as these substances may be secreted from the cells into membrane-bound extracellular vesicles, such as exosomes.

In one embodiment, the stem cells expressing the CD141 cell surface antigen may be derived from bone marrow, fat, blood, pulp or tonsil.

In one aspect, the present disclosure relates to a secretome secreted from stem cells expressing a CD141 cell surface antigen.

In one embodiment, the stem cells expressing the cell surface antigen CD141 may be CD141⁺Vasculogenic Multipotent Stem Cells (CD141⁺vMSCs).

In one embodiment, the stem cells expressing the cell surface antigen CD141 may be CD141⁺Vasculogenic Multipotent Stem Cells (CD141⁺vMSCs) deposited under the accession number KCLRF-BP-00524.

In one embodiment, the secretome may be included in a culture supernatant, a filtrate thereof, or a concentrate thereof obtained by culturing stem cells expressing the CD141 cell surface antigen in a serum-free medium containing a human platelet lysate.

In one embodiment, the secretome may be included in a culture supernatant obtained by culturing stem cells expressing the CD141 cell surface antigen in an FGF-free medium containing a human platelet lysate, heparin, EGF, ascorbic acid and hydrocortisone.

In one embodiment, the culture solution may be a culture solution in which the stem cells are cultured using a 2D culture method or a 3D culture method.

In one embodiment, the secretome may include Endoglin, HGF, IGFBP-2, IL-8, Pentraxin 3, TIMP-1 or uPA.

In one embodiment, the secretome may include an extracellular vesicle, protein, nucleic acid, a growth factor or cytokine secreted by the stem cell, wherein the extracellular vesicle may be an exosome, and the exosome may be an exosome having a size of 200 to 100 nm.

In one embodiment, when the secretomes of the present disclosure include the exosomes secreted by the stem cells, the culture solution may be filtered through a filter of 200 to 600 nm to remove apoptosomes (about 1000 to 600 nm in size), which are microbodies associated with cell death.

In one embodiment, the secretomes may increase HGF and decrease VEGF compared to secretomes secreted from bone marrow-derived mesenchymal stem cells.

In one embodiment, the secretome of the present disclosure may be used in combination with a drug delivery system, and the drug delivery system may be a liposome, an LNP, a hydrogel, or a biodegradable tissue engineering graft.

In one embodiment, the secretome of the present disclosure may be included or encapsulated in a nanoparticle.

The term "secretome" as used herein interchangeably refers to one or more molecules and/or biological factors secreted by the stem cells of the present disclosure into an extracellular space (e.g., culture solution). The secretome may include extracellular vesicles (e.g., exosomes, microparticles, etc.), proteins, nucleic acids, growth factors, cytokines, and/or other molecules secreted by the cells into the extracellular space (e.g., culture solution). The secretome includes a secretory proteome, which means the sum total of the protein components, and the secretory proteome means components released into an extracellular environment by the cells after undergoing transcription, translation, and post-translational modification of genes within the cells. The secretome may be left unpurified or may be further processed (e.g., components of the secretome may be present in the culture solution, or may be purified, isolated, and/or concentrated from the culture solution or an extract, portion, or fraction thereof). The secretome may further include one or more substances (e.g., culture solution, additives, nutrients, etc.) that are not secreted from the cell.

As used herein, the term "exosome" refers to a microbody that is secreted in various secretory forms depending on an environment of the cell from a special organ multivesicular endosome (MVE) within the cell, with a unit of approximately 30 to 100 nm, and includes various growth factors, cytokines, and nucleic acids (DNA and RNA (especially microRNA)), and has the structure as a liposome composed of a phospholipid membrane.

The term "isolated" as used herein means a substance that has been removed from its original environment, and thus is altered "by human hands" from its natural state.

The term "concentration" as used herein means selectively concentrating or increasing the amount of one or more components in a composition with respect to one or more other components. In some examples, the concentration may include reducing or lowering (e.g., removing or eradicating) the amount of an undesired substance and/or include specifically selecting or isolating a desired substance from the composition.

The term "stem cell" used herein refers to undifferentiated cells that may differentiate into various cells that constitute biological tissues and regenerate without limitation to form specialized cells of tissues and organs. The stem cells are pluripotent or multipotent cells capable of development. The stem cells proliferate into mature and complete cells of the tissue.

The term "mesenchymal stem cell" used herein refers to an undifferentiated cell having multipotency derived from an adult cell of a mammal including a human, preferably a human, and means a stem cell having multipotency capable of differentiating into adipocytes, bone cells, chondrocytes, muscle cells, nerve cells, and cardiomyocytes. The mesenchymal stem cells may be derived from various adult cells, including, for example, bone marrow, blood, brain, skin, fat (i.e., adipose tissue or adipocytes), umbilical cord blood, and umbilical Wharton's jelly.

The term "differentiation" used herein refers to a phenomenon in which less specialized cells develop into specific cells, and the size, shape, membrane potential, metabolic activity, and response to signals of the cells change into specific types of cells, and a state in which a qualitative difference occurs between parts of a certain biological system that were initially almost homogeneous, or as a result, the system is divided into qualitatively distinguishable subsystems. In particular, differentiation of the stem cell refers to a phenomenon in which stem cells develop in a direction into cells with a specific function.

The term "expression" as used herein generally refers to a cellular process by which a biologically active polypeptide is produced from a DNA sequence and exhibits biological activity in a cell. In that meaning, gene expression includes not only transcription and translation processes, but also post-transcriptional and post-translational processes that may affect the biological activity of a gene or gene product. The processes include RNA synthesis, processing and transport, as well as polypeptide synthesis, transport and post-translational modifications of polypeptides, but are not limited thereto.

The term "overexpression" used herein means that the expression of a specific gene into mRNA or the expression level of the gene into protein is significantly up-regulated through intracellular gene transcription or gene translation.

The term "not expressed" used herein means that the expression of a specific gene into mRNA or the expression level of the gene into protein is significantly "downregulated" by intracellular gene transcription or gene translation, so that there is almost no expression level.

In the present disclosure, the expression may be confirmed by measuring the expression level of the gene or mRNA by polymerase chain reaction (PCR), real-time RT-PCR, reverse transcription PCR, competitive RT-PCR, nuclease protection analysis (RNase, S1 nuclease assay), in situ hybridization, nucleic acid microarray, Northern blot, or DNA chip method using a primer pair, a probe, or a primer pair and a probe that specifically recognizes a nucleic acid sequence, a nucleic acid sequence complementary to the nucleic acid sequence, and fragments of the nucleic acid sequence and the complementary sequence. In addition, the expression may be confirmed by measuring the expression level of protein by Western blot, enzyme linked immunosorbent assay (ELISA), radioimmunoassay (RIA), radioimmunodiffusion, immunoelectrophoresis, immunohistochemical staining, immunoprecipitation assay, complement fixation assay, FACS, mass spectrometry, or protein microarray using antibodies, antibody fragments, aptamers, avidity multimers, or peptidomimetics that specifically recognize a full-length protein or fragments thereof.

In one aspect, the present disclosure relates to a pharmaceutical composition for vascular regeneration or vasculogenesis, including a culture solution of stem cells expressing a CD141 (thrombomodulin TM) cell surface antigen as an active ingredient.

In one embodiment, the vascular regeneration may include arterial regeneration, arteriolar regeneration, venous regeneration, capillary vascular regeneration, blood-brain barrier (BBB) regeneration, or retinal vascular regeneration.

In one embodiment, the culture solution of the stem cells expressing the CD141 cell surface antigen may be a culture or a supernatant thereof obtained during or after culturing the cells in a medium. The culture solution of the stem cells expressing the CD141 cell surface antigen may be a concentrate of a culture, a filtrate thereof, a fraction thereof, or a supernatant thereof, a lyophilized product thereof, or a supercritical dried product thereof obtained during or after culturing the cells. The concentrate may be obtained by concentrating a culture of stem cells expressing the CD141 cell surface antigen or a supernatant thereof about 2-fold, about 3-fold, about 4-fold, about 5-fold, about 6-fold, about 7-fold, about 8-fold, about 9-fold, about 10-fold, or about 20-fold, and may be obtained by removing apoptosomes and cell debris using a filter having MWCO of 600 nm or more, and concentrating the culture about 2-fold, about 3-fold, about 4-fold, about 5-fold, about 6-fold, about 7-fold, about 8-fold, about 9-fold, about 10-fold, or about 20-fold using an ultracentrifuge.

In one embodiment, the filtrate may be obtained by filtering the culture solution using a filter of 200 to 600 nm to remove apoptosomes (about 1000 to 600 nm in size), which are microbodies associated with cell death.

In one embodiment, the culture solution of the stem cells expressing the CD141 cell surface antigen may include secretomes secreted outside the cells by producing bioactive molecules through intercellular interactions during the process of culturing the cells. The secretomes may include substances such as cell-derived proteins, cytokines, growth factors, exosomes, nucleic acids (DNA and RNA), and the like, and various bioactive molecules such as these substances may be secreted from the cells into membrane-bound extracellular vesicles, such as exosomes.

In one embodiment, the secretome may include an extracellular vesicle, protein, nucleic acid, growth factor or cytokine secreted by the stem cell, and the extracellular vesicle may be an exosome.

In one embodiment, the stem cells (Vasculogenic Multipotent Stem Cells, vMSCs) expressing the CD141 cell surface antigen may be cells deposited under the accession number KCLRF-BP-00524.

In one embodiment, the culture solution may be a culture solution obtained by culturing the stem cells expressing the CD141 cell surface antigen in a serum-free medium.

In one embodiment, the composition may further include a culture solution of bone marrow-derived mesenchymal stem cells (BM-MSCs).

In one embodiment, the culture solution of the bone marrow-derived mesenchymal stem cells may be a culture or a supernatant thereof obtained during or after culturing the cells in a medium. The culture solution of the bone marrow-derived mesenchymal stem cells may be a concentrate of a culture, a fraction thereof, or a supernatant thereof, or a lyophilized product thereof obtained during or after the process of culturing the bone marrow-derived mesenchymal stem cells. The concentrate may be obtained by concentrating a culture of bone marrow-derived mesenchymal stem cells or a supernatant thereof about 2-fold, about 3-fold, about 4-fold, about 5-fold, about 6-fold, about 7-fold, about 8-fold, about 9-fold, about 10-fold, or about 20-fold. The bone marrow-derived mesenchymal stem cell culture solution may include cell-derived proteins, i.e., a secretomes, that produce and secrete useful proteins outside the cells through interactions between cells during the process of culturing the cells.

In one aspect, the present disclosure relates to a pharmaceutical composition for vascular regeneration or vasculogenesis, including secretomes secreted by stem cells expressing a CD141 cell surface antigen.

In one embodiment, the secretome secreted by the stem cells expressing the CD141 cell surface antigen may include Endoglin, HGF, IGFBP-2, IL-8, Pentraxin 3, TIMP-1 or uPA.

In one embodiment, the secretomes secreted by the stem cells expressing the CD141 cell surface antigen may increase HGF and decrease VEGF compared to secretomes secreted from bone marrow-derived mesenchymal stem cells.

In one embodiment, the composition may further include secretomes secreted from bone marrow-derived mesenchymal stem cells.

In one embodiment, the secretome secreted by the bone marrow-derived mesenchymal stem cells may include Angiogenin, Endoglin, HGF, IGFBP-2, IGFBP-3, IL-8, Pentraxin 3, TIMP-4, uPA or VEGF.

The term "vascular regeneration" used herein means repairing damage to damaged blood vessels, and means promoting angiogenesis, i.e., regeneration of damaged blood vessels, and preventing already damaged blood vessels from being further damaged by continuous external force, etc., using the composition of the present disclosure. The "angiogenesis" or "vasculogenesis" refers to a natural healing process of forming new blood vessels to supply blood to organs or damaged tissues, and the angiogenesis plays an important role in the progression and treatment of many diseases.

In one aspect, the present disclosure relates to a composition for promoting angiogenesis of stem cells, including a culture solution of stem cells expressing a CD141 cell surface antigen or secretomes secreted from the stem cells expressing the CD141 cell surface antigen.

In one aspect, the present disclosure relates to stem cells treated with a culture solution of stem cells expressing a CD141 cell surface antigen or secretomes secreted from the stem cells expressing the CD141 cell surface antigen.

In one embodiment, the stem cells may be cardiac progenitor cells (CPCs), endothelial progenitor cells (EPCs), endothelial colony forming cells (ECFCs), vasculogenic progenitor cells (VPCs), mesenchymal stem cells, embryonic stem cells or myoblasts. The mesenchymal stem cells may be umbilical cord blood-derived mesenchymal stem cells (UCB-MSCs), umbilical cord-derived mesenchymal stem cells (UC-MSCs), adipose-derived mesenchymal stem cells (AD-MSCs) or bone marrow-derived mesenchymal stem cells (BM-MSCs).

In one embodiment, the EPCs may be umbilical cord blood-derived EPCs.

In one embodiment, the stem cells may be stem cells expressing a CD141 cell surface antigen, i.e., CD141⁺vMSCs, and may be bone marrow or adipose-derived stem cells expressing a CD141 cell surface antigen.

In one embodiment, the stem cells may be stem cells with proangiogenic capacity enhanced by treatment with the culture solution or secretomes.

In one embodiment, the stem cells may be further treated with a culture solution of bone marrow-derived mesenchymal stem cells or secretomes secreted from the bone marrow-derived mesenchymal stem cells.

In one embodiment, the stem cells may have increased vascular sprouting ability, i.e., angiogenic ability, compared to stem cells that are not treated with the culture solution or secretomes.

In one embodiment, the stem cells may be 3D cultured stem cells.

In one aspect, the present disclosure relates to a cell therapy product composition for vascular regeneration or vasculogenesis, including the stem cells treated with the secretomes of the present disclosure or the culture solution an active ingredient.

In one embodiment, the cell therapy product composition may be frozen and stored and used for vascular regeneration.

The cell therapy product may be administered to the human body through any common route as long as it may reach a target tissue, and may be administered parenterally or orally, and when administered orally, may be administered by including the cells in a grafting material, etc.

The term "cell therapy product" used herein refers to a therapy product used for tissue regeneration treatment by proliferating and selecting living autologous, allogenic, or xenogenic stem cells *in vitro* and introducing the stem cells into the body to restore the tissue and function of cells.

In one aspect, the present disclosure relates to a graft material for vascular regeneration, including, as an active ingredient, a stem cell treated with a secretome secreted from a stem cell expressing a CD141 cell surface antigen or a cell therapy product composition including the stem cell.

In one embodiment, the graft material may be surgical implants, medical supplies or medical devices, such as film, membrane, sheet, rod, screw, anchor, pin, implant, stent, surgical suture, scaffold for tissue regeneration, bio-nano fiber, hydrogel, bio-sponge, bone plate and bone graft.

In one embodiment, if the graft material is a hydrogel, the graft material may be administered orally and used for promoting vascular regeneration of internal organs.

In one embodiment, the graft material may further include a biodegradable polymer.

In one embodiment, the graft material may be a composite scaffold for tissue engineering, and the composite scaffold for tissue engineering may include stem cells of the present disclosure or a cell therapy product composition of the present disclosure in a scaffold made by molding a biodegradable polymer.

In one embodiment, the graft material may be a graft material in which a stem cell treated with a secretome secreted from a stem cell expressing the CD141 cell surface antigen of the present disclosure or a cell therapy product including the same is inoculated onto a composite support for tissue engineering.

In one embodiment, when the graft material is a hydrogel, the polymer forming the hydrogel may be polyethylene glycol (PEG), polyethylene oxide (PEO), polyhydroxyethyl methacrylate (PHEMA), polyacrylic acid (PAA), polyvinyl alcohol (PVA), poly(N-isopropylacrylamide) (PNIPAM), polyvinylpyrrolidone (PVP), polylactic acid (PLA), polyglycolic acid (PGA), polycaprolactone (PCL), gelatin, hyaluronic acid, alginate, carrageenan, chitosan, hydroxyalkyl cellulose, alkyl cellulose, silicone, rubber, agar, carboxyvinyl copolymer, polydioxolane, polyacryl acetate, polyvinyl chloride, or maleic anhydride/vinyl ether.

The term "biodegradable polymer" used herein refers to a polymer that is slowly and spontaneously decomposed in a living body after a certain period of time, and has at least one property of biocompatibility, hemocompatibility, anti-calcification properties, cellular nutrients, and intercellular matrix forming ability. The type of such biodegradable polymer is not particularly limited in the present disclosure, but representative examples thereof may include fibrin, collagen, gelatin, chitosan, alginate, hyaluronic acid, dextran, polylactic acid, poly(glycolic acid) (PGA), poly(lactic-co-glycolic acid) (PLGA), poly-ε-(caprolactone), polyanhydride, polyorthoester, polyvinyl alcohol, polyethylene glycol, polyurethane, polyacrylic acid, poly-N-isopropylacrylamide, poly(ethylene oxide)-poly(propylene oxide)-poly(ethylene oxide) copolymer, copolymers thereof, mixtures thereof, etc. The composite support may be prepared by molding a biodegradable polymer according to a conventionally known method, for example, a solvent-casting and particle-leaching technique, a gas forming technique, a fiber extrusion and fabric forming process, a thermally induced phase separation technique, an emulsion freeze drying method, a high-pressure gas expansion method, etc.

In one aspect, the present disclosure relates to a pharmaceutical composition for preventing or treating vascular diseases or vascular dysfunction, including, as an active ingredient, secretomes secreted from stem cells expressing the CD141 cell surface antigen of the present disclosure or a culture solution, or stem cells of the present disclosure treated with the secretomes or the culture solution.

In one embodiment, the vascular diseases may be traumatic vascular injury, peripheral vascular disease, cardiovascular disease, cerebrovascular disease, or ischemic disease, and the ischemic disease may be ischemic myocardial infarction, ischemic heart disease, ischemic vascular disease, ischemic enteritis, ischemic eye disease, ischemic glaucoma, ischemic renal failure, ischemic retinopathy, ischemic stroke, or ischemic lower extremity disease.

In one embodiment, the vascular disease or vascular dysfunction may be critical limb ischemia, diabetic angiogenic disorders, vascular dementia, diabetic organ damage, arteriosclerosis, angina, peripheral cardiovascular disease, hypertension, cerebral infarction, brain injury sequelae, heart failure, peripheral circulatory disorders, myocardial infarction, arterial occlusive disease, stroke, spinal cord injury, spinal nerve sequelae, degenerative disease, cerebral infarction sequelae, peripheral neuropathy, diabetic ulcer, presbyopia, degenerative hearing loss, brain surgery sequelae, ischemic stroke, or subarachnoid hemorrhage.

In one embodiment, the composition may induce angiogenesis or vasculogenesis and promote vascular regeneration or tissue regeneration, or improve vascular function and increase tissue perfusion and new blood vessel formation.

In one aspect, the present disclosure relates to a cell therapy product composition for treating brain nervous system diseases, including, as an active ingredient, secretomes secreted from stem cells expressing a CD141 cell surface antigen of the present disclosure, a culture solution or stem cells of the present disclosure treated with the secretomes or the culture solution.

In one embodiment, the brain nervous system diseases may be a degenerative brain disease, a mental disorder, or a developmental disorder.

In one embodiment, the degenerative brain disease may be cognitive impairment, Alzheimer's disease, dementia with Lewy bodies, frontotemporal dementia, Parkinson's disease, Creutzfeldt-Jakob disease (CJD), Huntington's disease, multiple sclerosis, or Guillain-Barre Syndrome (GBS).

In one embodiment, the mental disorder may be bipolar disorder, autism, depression, hyperactivity, attention deficit, post-traumatic stress disorder (PTSD), anxiety disorder, sleep disorder, panic disorder, intellectual disability, memory impairment, drug addiction, schizophrenia, obsessive-compulsive disorder, delusions of grandeur, personality disorder, alcoholism, or manic depression.

In one embodiment, the developmental disorder may be epilepsy, cerebral palsy, developmental language disorder, disturbance of sense, learning disorder, attention deficit hyperactivity disorder (ADHD), autism spectrum disorder (ASD), intellectual disability, cognitive impairment, or impulse control disorders.

The pharmaceutical composition of the present disclosure is administered in a pharmaceutically effective amount. As used herein, the term 'pharmaceutically effective dose' refers to an amount enough to treat the disease at a reasonable benefit/risk ratio applicable to medical treatment and not to cause side effects. An effective dose level may be determined according to factors including the health condition of an individual, the type of vascular diseases or vascular dysfunction, the severity, the activity of a drug, the sensitivity to a drug, a method of administration, a time of administration, a route of administration, an emission rate, duration of treatment, the combined or simultaneously used drugs, and other factors well-known in the medical field. The composition of the present disclosure may be administered as an individual therapy product or in combination with other therapy products, and may be administered sequentially or simultaneously with conventional therapy products, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include a carrier, a diluent, an excipient, or a combination of two or more thereof, which are commonly used in biological agents. As used herein, the term "pharmaceutically acceptable" means exhibiting non-toxic properties to cells or humans exposed to the composition. The carrier is not particularly limited as long as the carrier is suitable for in vivo delivery of the composition, and may be used by mixing, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present disclosure may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by mixing, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present disclosure may further contain one or more active ingredients exhibiting the same or similar functions.

The pharmaceutical composition of the present disclosure may further include a pharmaceutically acceptable additive. At this time, the pharmaceutically acceptable additive may be used with starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present disclosure is preferably included in an amount of 0.1 part by weight to 90 parts by weight based on the composition, but is not limited thereto.

The composition of the present disclosure may be administered parenterally (e.g., applied intravenously, subcutaneously, intraperitoneally or topically) or orally according to a desired method, and the range of the dose may vary depending on the weight, age, sex, and health condition of a patient, a diet, an administration time, an administration method, an excretion rate, the severity of a disease, etc. A daily dose of the composition according to the present disclosure is 0.0001 to 10 mg/ml, preferably 0.0001 to 5 mg/ml, and more preferably administered once to several times a day.

Liquid formulations for oral administration of the composition of the present disclosure correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In one aspect, the present disclosure relates to a method for producing vMSC-derived secretomes, including culturing stem cells expressing a CD141 cell surface antigen in a serum-free medium containing a human platelet lysate.

In one embodiment, the method may further include collecting a culture solution after the culturing and centrifuging the collected culture solution to collect a culture supernatant.

In one embodiment, the method may further include filtering and concentrating the collected culture supernatant after collecting the culture supernatant.

In one embodiment, the filtering may be performed through a filter of 200 to 600 nm to remove apoptosomes.

In one embodiment, the removing of the apoptosomes may be performed by filtration through a filter having a 200 nm MWCO or centrifugation at 10,000 to 15,000 g.

In one embodiment, the method may further include freeze-drying or supercritical-drying.

In one embodiment, the method may further include dialyzing the collected culture supernatant after collecting the culture supernatant.

In one embodiment, the concentration of HGF as a marker substance may be monitored during the process of producing the vMSC-derived secretomes.

In one aspect, the present disclosure relates to a use of secretomes secreted from stem cells expressing a CD141 cell surface antigen, for use in vascular regeneration or vasculogenesis.

In one aspect, the present disclosure relates to a use for vascular regeneration or vasculogenesis of stem cells treated with secretomes secreted from stem cells expressing a CD141 cell surface antigen or a culture solution of the stem cells expressing the CD141 cell surface antigen.

In one aspect, the present disclosure relates to a method for treating vascular diseases or vascular dysfunction, including administering, to a subject having vascular diseases or vascular dysfunction, secretomes secreted from stem cells expressing a CD141 cell surface antigen or a culture solution of the stem cells expressing the CD141 cell surface antigen.

In one aspect, the present disclosure relates to a method for preventing or treating brain nervous system diseases, including administering, to a subject having brain nervous system diseases, secretomes secreted from stem cells expressing a CD141 cell surface antigen or a culture solution of the stem cells expressing the CD141 cell surface antigen.

### [Modes of the Invention]

Hereinafter, the present disclosure will be described in more detail with reference to the following Examples. However, the following Examples are only intended to embody the contents of the present disclosure, and the present disclosure is not limited thereto.

### Example 1. Isolation of novel multipotent stem cells derived from bone marrow

### 1-1. Optimal culture of vMSCs

Cryopreserved human bone marrow mononuclear cells (BM-MNCs) (STEMCELL Technologies, Vancouver, Canada, LONZA, Basel, Switzerland) were thawed at 37°C, or a bone marrow aspirate obtained with patient consent was subjected to density gradient centrifugation with Ficoll-paque (Cytiva, MA, USA) to obtain BM-MNCs. The BM-MNCs obtained above were seeded at a density of 1.0 to 2.0 × 10⁵ cells/cm² in a TC-treated (tissue culture treated) flask using an FBS (Fetal bovine serum)-deficient EGM-2 bulletkit medium (Lonza; Basel, Switzerland) (EGM-2-FBS+2%hPL) supplemented with 2% human platelet lysate (PL bioscience, GmbH, Germany) and 2 unit/mL heparin (Sigma-Aldrich, St. Louis), and the primary-cultured for 7 to 12 days. During the primary culture period, medium replacement was performed once every 1 to 4 days. Colonies of the novel multipotent stem cells of the present disclosure, Vasculogenic Multipotent Stem Cells (vMSCs), begin to be observed from 3 to 5 days of culture, and the vMSCs cells forming the colonies observed during the primary culture had a spindle shape and were very diverse in length and size, and it was observed that the colonies expanded significantly between 7 and 10 days. The first subculture period of vMSCs was performed when the majority of colonies showed a density of 80 to 90% or more, and the vMSCs were isolated into single cells using trypsin or a trypsin substitute in the same manner as the subculture method of general adherent cells, and then the isolated cells were dispensed into a new flask at 1.0 to 5.0 × 10³ cells/cm² and subcultured when the density was 70 to 90%, and then subcultured 5 to 7 times. As such, the vMSCs of the present disclosure were cultured by excluding FBS from EGM-2 and adding a human PL (human platelet lysate), unlike endothelial progenitor cells (EPCs) that were cultured with EGM-2 containing FBS using basic components of an EGM-2 kit.

### 1-2. Confirmation of extracellular matrix adhesion properties of vMSCs

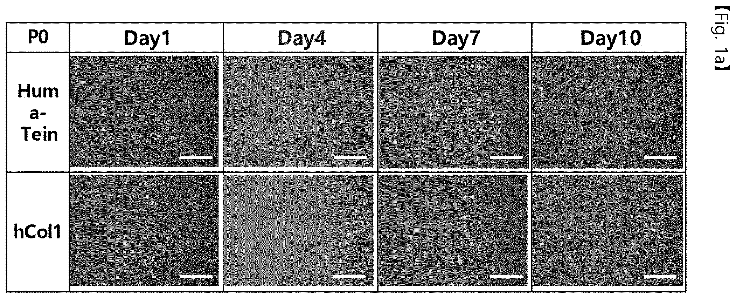

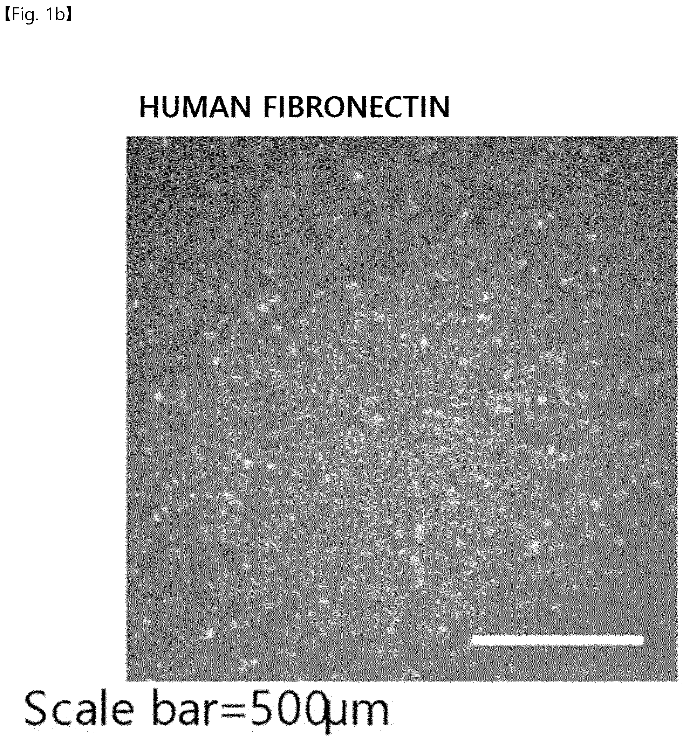

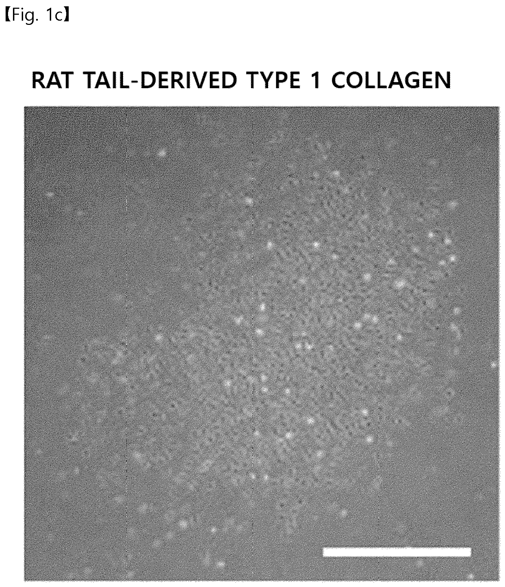

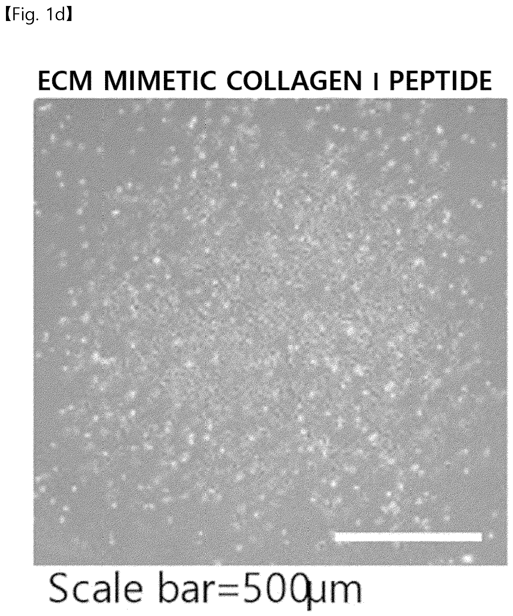

The vMSCs were cultured on a culture flask coated with various extracellular matrices (ECMs), including a human fibroblast-derived ECM complex, HumaTein (100 µg/mL, ROKIT healthcare, Seoul, Republic of Korea), human type I collagen (0.2 µg/cm²) (Corning, NY, USA), Fibronectin (1 µg/cm²) (Sigma-Aldrich), porcine collagen type 1-P (Cellmatrix Type I-P) (Nitta Gelatin, Osaka, Japan), collagen type I from rat tail (50-825 µg/mL) (ThremoFisher, MA, USA), and collagen type I peptide (Corning). As a result, the cells forming the vMSC colonies were identically shown in a spindle shape on all coating materials, and could be cultured in various extracellular matrices. When the cells were cultured in the ECM-coated flask as described above, more colonies were obtained than in a general TC-treated flask, but there was no significant difference (FIG. 1). That is, although the primary culture yield may vary depending on the presence and type of ECM coating, there was little difference in the proliferation rate after P1 depending on the type and presence of coating.

The novel vMSCs of the present disclosure may not maintain the characteristics of CD141+vMSCs when cultured using FBS of an existing EGM-2 bullet kit instead of human PL, and the addition of human PL further increased the expression of CD141. Therefore, the culture solution thereof essentially added human PL to an EGM-2 bullet kit (Lonza) and excluded FBS, and there was no problem with growth even if IGF and VEGF were excluded, and thus IGF and VEGF may be excluded.

### Example 2. Marker characteristic analysis of vMSCs

### 2-1. Confirmation of expression of EPC and vascular endothelial cell markers

The expression of markers of conventional EPC and vascular endothelial cells in vMSCs of the present disclosure was confirmed by flow cytometry, and the expression of eNOS and VE-cadherin expressed by mature vascular endothelial cells was confirmed by immunofluorescence staining analysis, and compared with those of bone marrow mononuclear cells (BM-MSCs) and human umbilical cord endothelial cells (HUVECs), respectively. Specifically, for flow cytometry, vMSCs and BM-MSCs were resuspended in a PEB buffer containing 2 mM EDTA and 0.5% bovine serum albumin (BSA) (Sigma-Aldrich), respectively, and then blocked with a human FcR blocking reagent (Miltynyi biotec) at 4°C for 10 minutes. Thereafter, the vMSCs and BM-MSCs were stained with Alexa flour 488-conjugated vWF antibody (Abcam, 1:500), fluorescein-labeled UEA-1 (Vectorlabs, 3:100), and allophycocyanin (APC)-conjugated CD29, CD31, CD34, CD44, CD45, CD73, CD90, CD105, and CD309 antibodies (Miltenyl Biotec, 1:50), respectively. At this time, Isotype IgG conjugated with APC (Miltenyl Biotec. 1:50) and Alexa flour 488 (Abcam, 1:500) was used as a control group. After antibody staining, the cells were washed with a PEB buffer and centrifuged at 1,000 ×g and 4°C for 5 minutes. Finally, the cells were resuspended in a PEB buffer and analyzed using a NovoCyte 3000 flow cytometer (Agilent Technologies, Santa Clara, CA, USA) and NovoExpress software. In addition, for immunofluorescence staining analysis, vMSCs and HUVECs were cultured on coverslips, respectively, and fixed with 3.7% formaldehyde (Sigma-Aldrich), and then permeabilized into the cell membrane with 0.2% Triton X-100. For blocking, the membrane reacted with 20% normal goat serum (NGS) for 1 hour at room temperature, and then reacted with primary antibodies, e-NOS (Cell Signaling Technology, MA, USA, 1:400) and VE-cadherin (Cell Signaling Technology, 1:400), diluted in 20% NGS, at 4°C overnight, respectively. The next day, the membrane reacted with an Alexa fluor 488-conjugated anti-rabbit secondary antibody (Invitrogen, MA, USA, 1:1000) diluted in 20% NGS for 1 hour at room temperature. After mounting with a mounting solution containing DAPI (Vector Laboratories, CA, USA) for sample observation, images were obtained using a fluorescence microscope (Leica).

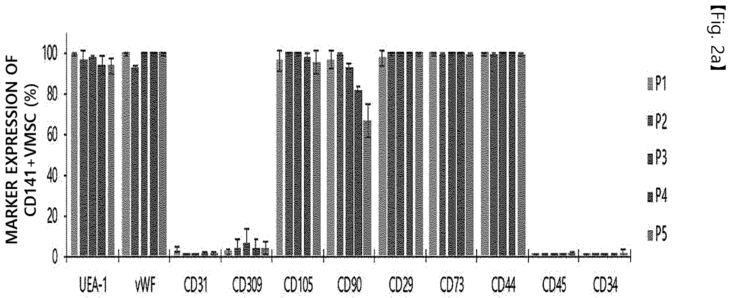

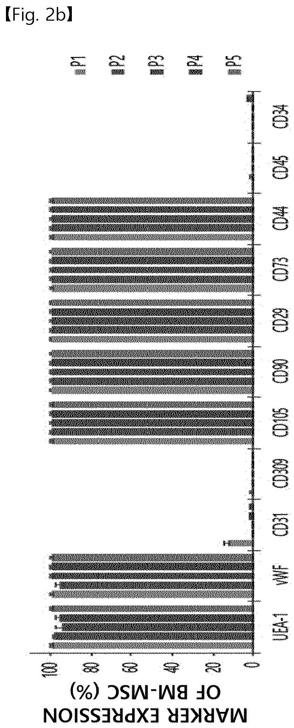

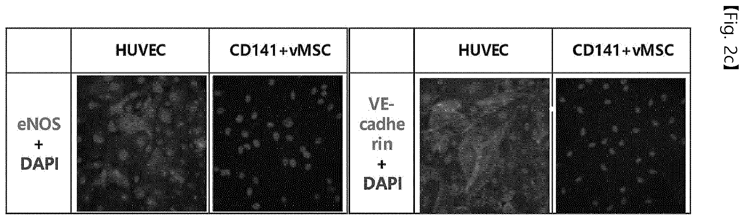

As the flow cytometry result, it was shown that the vMSCs of the present disclosure expressed all of CD105, CD90, CD29, CD73, and CD44, which were known as MSC markers, and did not express CD31, CD309, and CD34, which were commonly expressed by previously reported EPCs (FIG. 2A). In addition, as the result of immunofluorescence staining analysis, it was shown that the vMSCs of the present disclosure did not express eNOS and VE-cadherin, unlike HUVECs, as a positive control group (FIG. 2C). Through this, it may be seen that vMSCs share more markers with MSCs than EPCs.

### 2-2. Discovery of novel vMSC-specific markers

Marker screening was conducted to discover vMSC-specific markers of the present disclosure that were distinguished from BM-MSCs. To this end, MACS^{®}Marker Screen, human, version 02 (Miltenyi Biotec), capable of analyzing 378 surface antigen markers by flow cytometry, was used, and the experiment was performed according to the manufacturer's instructions. Specifically, vMSCs (5.7 × 10⁷) and BM-MSCs (5.7 × 10⁷) at Passage 3 derived from the same donor were resuspended in a PEB buffer and then blocked with a human FcR blocking reagent (Miltenyi Biotec) at 4°C for 10 minutes. Thereafter, 1.2 × 10⁵ /well of cells were dispensed into each of four 96-well plates containing six isotype controls and 378 antibodies, and then reacted at 4°C for 20 to 30 minutes. The cells were washed with a PEB buffer, centrifuged at 300 × g for 5 minutes, resuspended in PEB buffer at a concentration of 200 µl/well, and analyzed using a NovoCyte 3000 flow cytometer (Agilent Technologies) equipped with a NovoSampler (Agilent Technologies) and NovoExpress software.

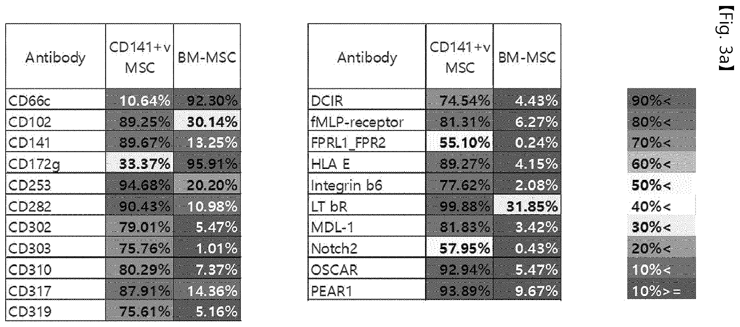

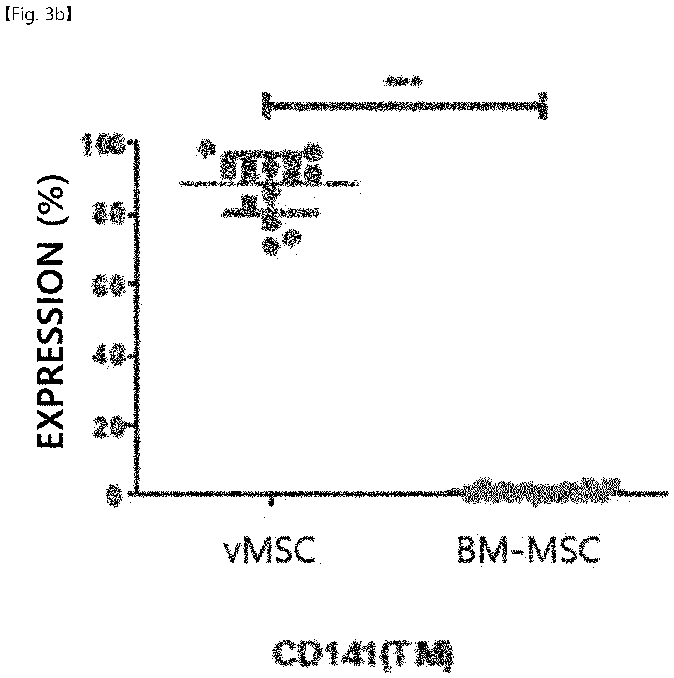

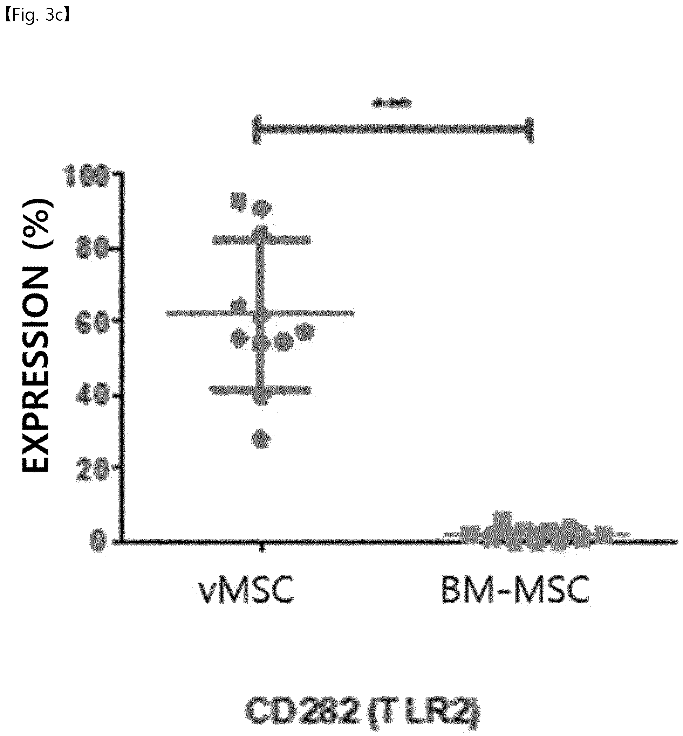

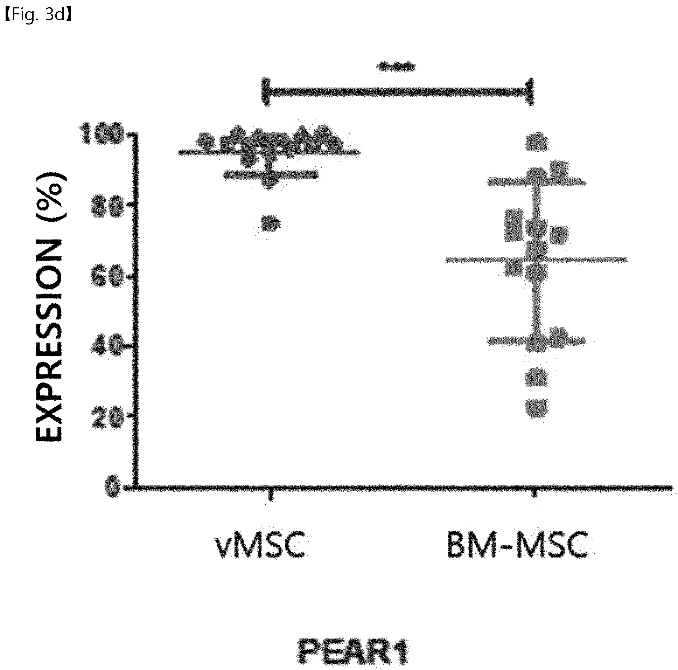

As a result of marker screening, about 21 markers that showed a difference between vMSCs and BM-MSCs were selected (FIG. 3A), and for validation, flow cytometry was performed for CD141, CD282, and PEAR1 (Platelet Endothelial Aggregation Receptor 1) in vMSCs and BM-MSCs derived from at least 10 donors using APC-conjugated CD141, CD282, and PEAR1 (Miltenyi biotec. 1:50). As a result, CD141 was consistently shown to be expressed at 70 to 99% in vMSCs, but not expressed in BM-MSCs (FIG. 3B). In addition, CD282 was not expressed in BM-MSCs, but was expressed in vMSCs at levels from 30% to 90%, showing a large variation between donors (FIG. 3C). In addition, PEAR1 was expressed at 70 to 99% in vMSCs, but expressed at levels from 20% to 90% in BM-MSCs, showing a large difference between the donors (FIG. 3D). Through this, it was shown that CD141 was a clear marker that distinguished between vMSCs and BM-MSCs of the present disclosure.

As shown in the results, the stem cells CD141⁺vMSC with multipotent and vascularization promoting ability identified in the present disclosure had the characteristics of not expressing CD31, CD309, and CD34 unlike EPC, not expressing eNOS and VE-cadherin unlike vascular endothelial cells, and expressing CD141 unlike mesenchymal stem cells. Therefore, the CD141⁺vMSCs were named CD141⁺Vasculogenic Multipotent Stem Cells and deposited with the Korea Cell Line Research Foundation (KCLRF) and assigned the accession number KCLRFBP00524.

### Example 3. Analysis of secretomes of vMSCs

### 3-1. Human Angiogenesis Array

To analyze the secretomes secreted by vMSCs (CD141⁺vMSCs) or BM-MSCs, bone marrow-derived vMSCs (CD141⁺vMSCs) and bone marrow-derived MSCs (BM-MSCs) were cultured using the culture method of Example above, respectively, and Human Angiogenesis Array was performed using the secretomes. Specifically, vMSCs and BM-MSCs of Passage 3 were dispensed at 1.0 × 10⁵ cells in T-75 flasks, respectively, and cultured in an EGM2-2%FBS+2%hPL medium and a StemMACS^{™} MSCS expansion medium, respectively, and then the cell culture supernatants were collected on day 4 of culture. Thereafter, Human Angiogenesis Array (R&D Systems, ARY007, Minneapolis, USA) was performed according to the manufacturer's instructions. As a control group, each culture medium (culture medium for BM-MSCs: StemMACS^{™} MSCS expansion media, and culture medium for vMSCs: EGM2-2%FBS+2%hPL) was used, and factors that significantly increased in the vMSC culture supernatant or the BM-MSC culture supernatant compared to the control group were identified. The blots of the analysis results were quantitatively analyzed using a protein array analyzer of the Image J program.

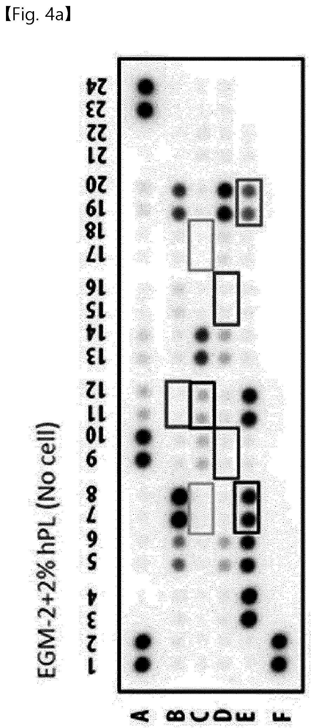

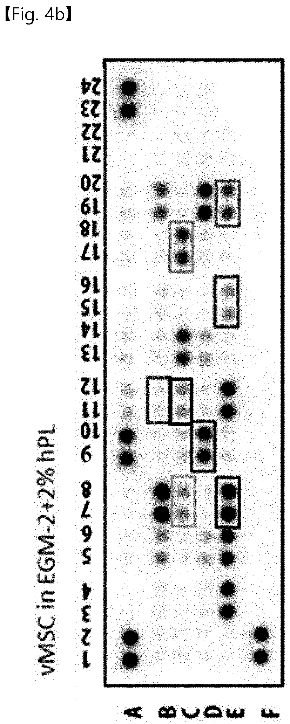

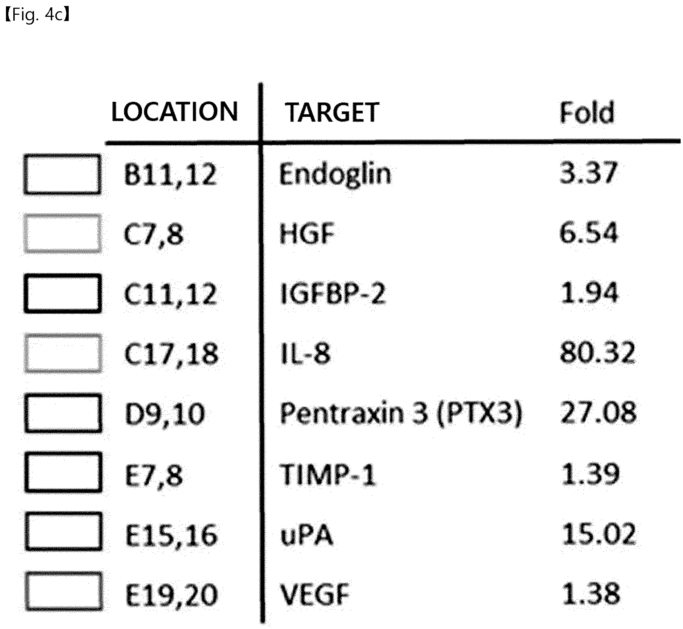

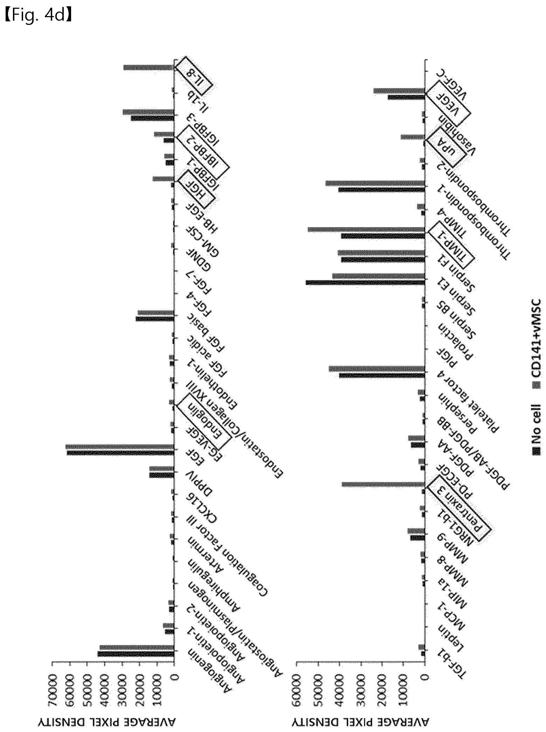

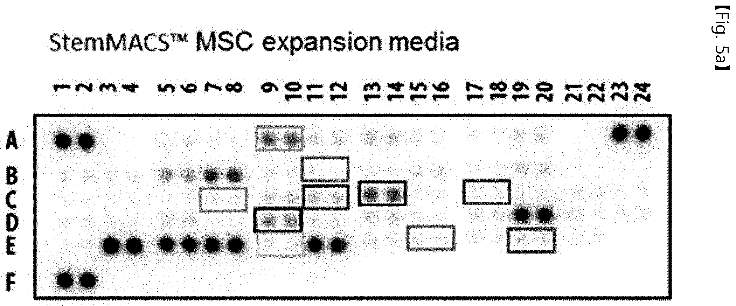

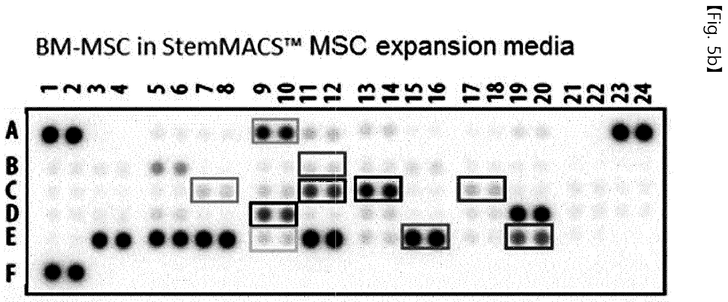

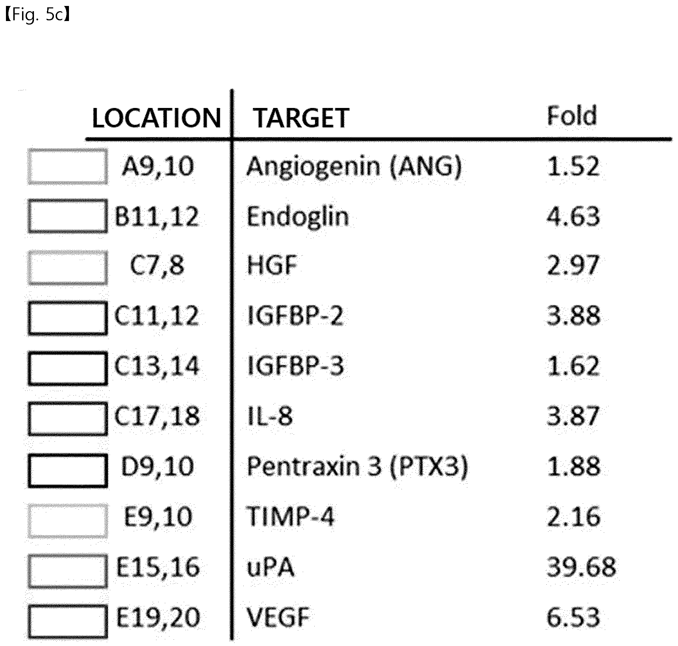

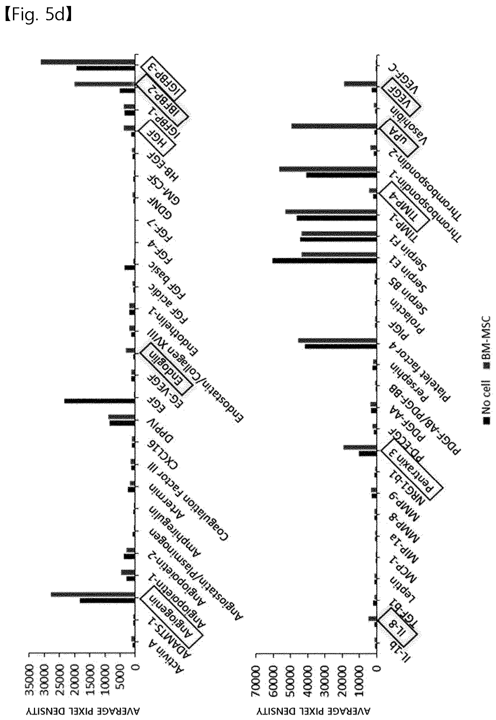

As a result, Endoglin, HGF, IGFBP-2, IL-8, Pentraxin 3, TIMP-1, and uPA were shown at significantly higher levels in the culture supernatant of vMSCs (CD141⁺vMSC) than in a control culture medium, and in particular, HGF was shown to be 6.5 times higher, IL-8 was shown to be 80.3 times higher, Pentraxin 3 was shown to be 27.1 times higher, and uPA was shown to be 15 times higher (FIG. 4). In addition, Angiogenin, Endoglin, HGF, IGFBP-2, IGFBP-3, IL-8, Pentraxin 3, TIMP-4, uPA, and VEGF were shown at significantly higher levels in the culture supernatant of BM-MSCs than in the control culture medium, and in particular, Endoglin was shown to be 4.6 times higher, IBFBP-2 and IL-8 were shown to be 3.9 times higher, uPA was shown to be 39.7 times higher, and VEGF was shown to be 6.5 times higher (FIG. 5).

### 3-2. ELISA analysis

To confirm the levels of HGF and VEGF among factors involved in angiogenesis in the secretomes secreted by vMSCs (CD141⁺vMSCs) or BM-MSCs confirmed in Example 3-1 above, vMSCs (1.3 × 10⁵) and BM-MSCs (2 × 10⁵) were cultured as in Example 3-1 above, and the cell culture supernatants were collected on day 4 or 5 of culture, respectively, and the cells were counted to reflect the cell number. In addition, to determine the secretion levels of HGF and VEGF when vMSCs and BM-MSCs were cultured under the same growth factor-deficient culture conditions, vMSCs and BM-MSCs at Passage 3 were dispensed at 9 × 10⁴/well in 6-well plates, respectively, and cultured in MEMα (Gibco, NY, USA) containing 0.2% hPL (PL BioScience, Aachen, Germany) as a growth factor-deficient medium. After replacing the medium with new medium on day 1, the cell culture supernatant was collected on day 4. Thereafter, Quantikine ELISA (R&D systems, Minneapolis, USA) was performed according to the manufacturer's instructions, and the absorbance was measured at 450 nm using a microplate reader (Molecular Device, SpectraMax ABS), and the levels of HGF and VEGF were analyzed by ELISA.

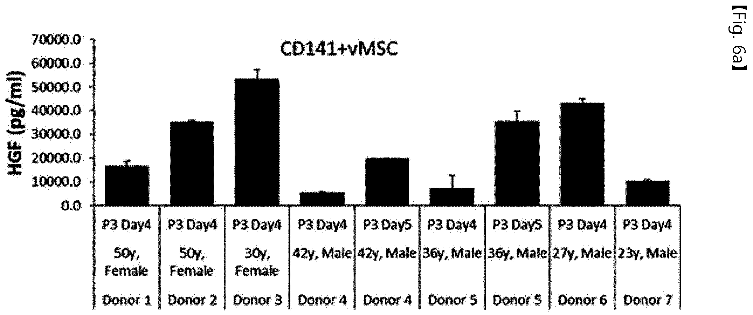

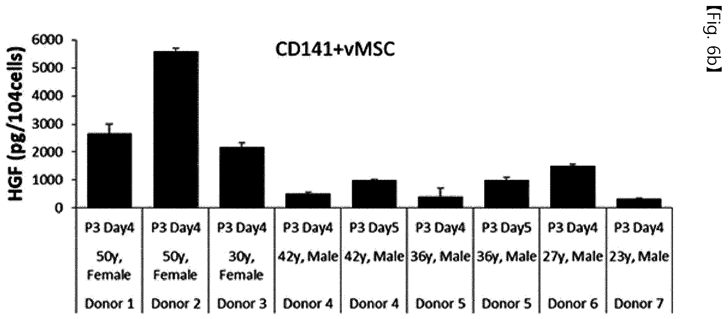

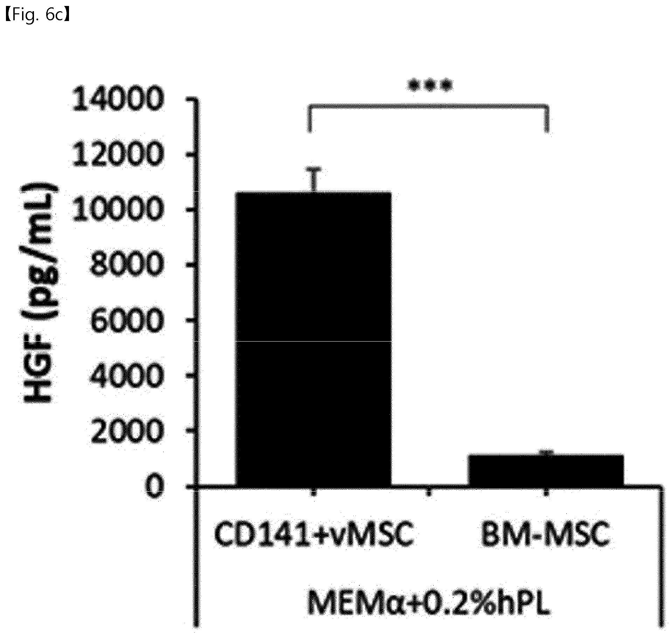

As the ELISA analysis result of HGF, it was shown that 5,400 pg/mL to 53,600 pg/mL (average 25,200 pg/mL) of HGF was measured in the culture supernatant of vMSCs (FIG. 6A). As the result of confirming the amount of HGF secreted per cell by dividing the HGF concentration by the cell number, it was found that vMSCs secreted 340 pg to 5,600 pg (average 1,700 pg) of HGF per 10⁴ cells (FIG. 6B). In addition, when vMSCs and BM-MSCs were cultured under the same conditions using a growth factor-deficient medium, HGF was measured at 10,600 pg/mL in the culture supernatant of vMSCs, and at 1,100 pg/mL in the culture supernatant of BM-MSCs, which was approximately 9 times lower than that in the culture supernatant of vMSCs (FIG. 6C).

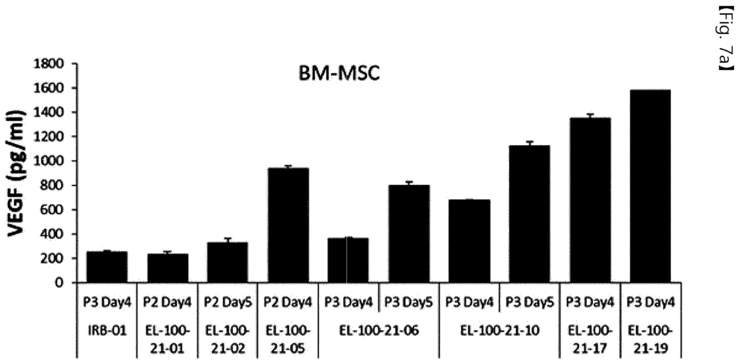

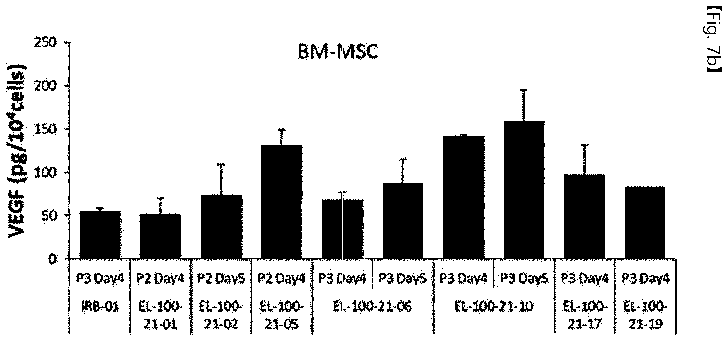

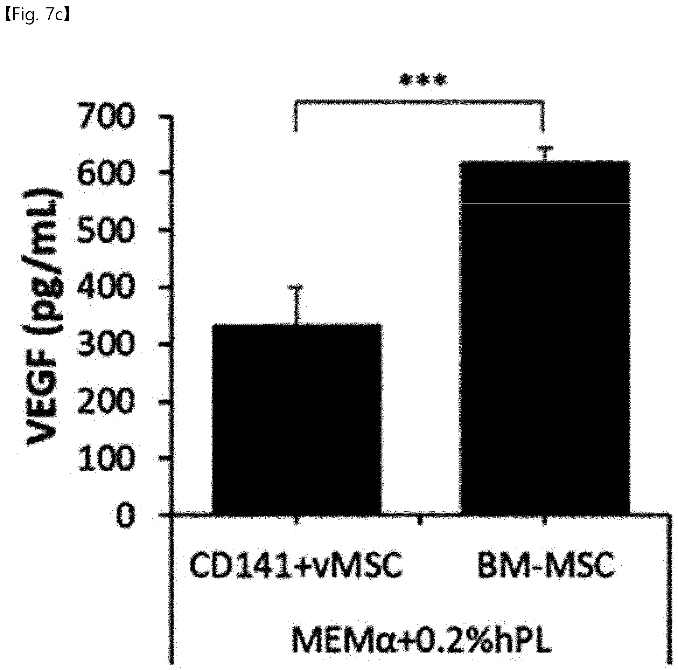

In addition, as the ELISA analysis result of VEGF, it was shown that 200 pg/mL to 1,600 pg/mL (average 820 pg/mL) of VEGF was measured in the culture supernatant of BM-MSCs (FIG. 7A), and as the result of confirming the amount of VEGF secreted per cell, it was shown that 55 pg to 160 pg (average 100 pg) of VEGF per 10⁴ cells was secreted (FIG. 7B). In addition, when vMSCs and BM-MSCs were cultured under the same conditions using a growth factor-deficient medium, VEGF was measured at 330 pg/mL in the culture supernatant of vMSCs, and at 620 pg/mL in the culture supernatant of BM-MSCs, which was approximately 2 times higher than that in the culture supernatant of vMSCs (FIG. 7C).

Through this, it was confirmed that vMSCs (CD141 positive cells) were cells that secreted HGF in a high concentration, and BM-MSCs were cells that secreted VEGF in a high concentration. In addition, it was confirmed that vMSCs secreted very high levels of not only HGF but also IL-8, Pentraxin 3, and uPA involved in angiogenesis, and that BM-MSCs were cells that secreted very high levels of not only VEGF but also uPA.

### Example 4. Effect of promoting cell sprouting of vascular endothelial cells by vMSC secretomes

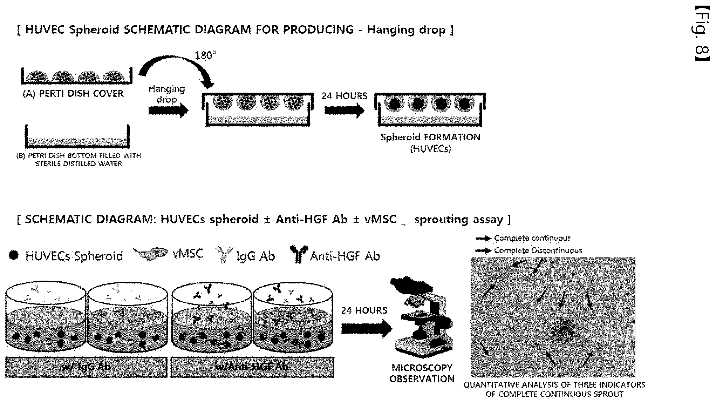

To confirm an effect of vMSC secretomes on promoting vascularization of vascular endothelial cells, HUVEC cells were cultured into spheroids using a hanging drop method, and non-contact co-cultured with vMSCs, and then spheroid sprouting assay was performed. Specifically, HUVECs were suspended at 1.33 × 10⁴ cells/mL in an M199 medium (Sigma-Aldrich, St. Louis, MO, USA) containing 10% FBS and 0.2% methylcellulose, and 30 µl of the suspended cells were dispensed onto the cover of a petri dish. To maintain humidity, sterile distilled water was placed at the bottom of the petri dish, the petri dish cover was turned over 180° and placed on the bottom of the petri dish containing sterile water, and HUVEC cells were cultured for 24 hours under conditions of 37°C and 5% CO₂ to form spheroids (Hanging drop in FIG. 8). Thereafter, HUVEC spheroids were suspended in M199 medium containing 20% FBS and 0.95% methylcellulose, and a Type I collagen solution, which was mixed with 30 mg/mL of type I-A collagen Gelatin, 10x Medium (Sigma-Aldrich), and 10x reconstitution buffer (0.05 N NaOH, 0.261 M NaHCO₃, and 0.2 M HEPES) in a ratio of 8:1:1 (v/v), and the suspended spheroids were mixed in a ratio of 1:1 (v/v). 0.7 mL of the mixture was dispensed into a 24-well plate, and then polymerized for 30 minutes under conditions of 37°C and 5% CO₂. The vMSCs diluted in 100 µl of M199 medium were dispensed at 1 × 10⁵ on collagen gel containing the polymerized HUVEC spheroids and cultured for 24 hours. At this time, to neutralize HGF among the secretomes of vMSCs, 500 ng/mL of HGF neutralizing antibody (MAB294, R&D systems; Anti-HGF Ab) was mixed in the M199 medium and polymerized, and 100 µl of the mixture was treated on the collagen gel containing the HUVEC spheroids and cultured for 24 hours, and mouse IgG (IgG) (500 ng/mL) was used as a control group (sprouting assay in FIG. 8). Sprouting images of spheroids were obtained by photographing with a microscope (Nikon), and the number of sprouts, cumulative sprouting length (sum of all sprout lengths formed from one spheroid), and average sprouting length (average of all sprout lengths formed from all spheroids) were quantitatively analyzed using Image J software (NIH).

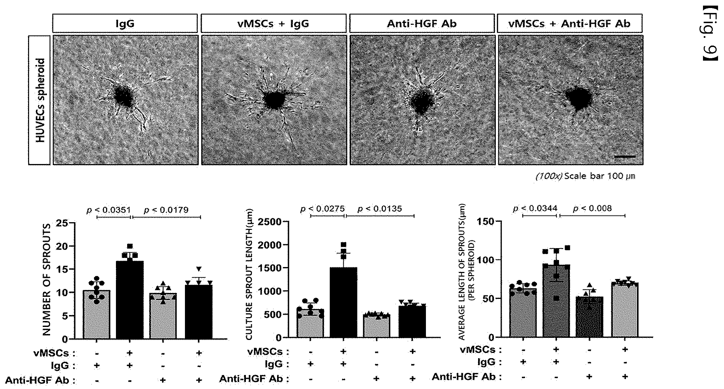

As a result, it was shown that a sprouting-promoting effect on HUVEC spheroids was exhibited during non-contact co-culture with CD141⁺vMSCs, and that the HUVEC sprouting effect was inhibited by treatment with HGF-neutralizing antibodies (FIG. 9). Through this, it was confirmed that HGF among the secretomes of vMSCs was a substance that exhibited a cell sprouting effect on vascular endothelial cells (HUVECs), i.e., promoted vascularization, and it may be determined that HGF is not sufficient for promoting HUVEC sprouting, but HGF is required for promoting HUVEC sprouting by vMSCs.

Therefore, bone marrow-derived vMSCs secreted an excessive amount of HGF, which was related to angiogenesis, and the secretomes promoted the vascular sprouting in HUVECs. Therefore, the secretome isolated by culturing vMSCs in a serum-free culture medium may be used to promote vascular regeneration.

### Example 5. Autocrine effect of vMSC secretomes

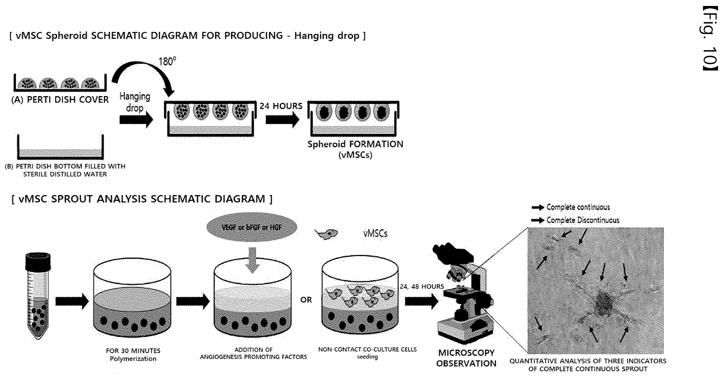

To confirm an effect of vMSCs on promoting vascularization by secretomes of vMSCs, i.e., an autocrine effect, vMSC cells were cultured into spheroids using the hanging drop method, and after non-contact co-culture with vMSCs, spheroid sprouting analysis was performed. Specifically, vMSCs were suspended at 2 × 10⁴ cells/mL in an M199 medium (Sigma-Aldrich, St. Louis, MO, USA) containing 10% FBS and 0.2% methylcellulose, and 30 µl of the suspended cells were dispensed onto the cover of a petri dish. To maintain humidity, sterile distilled water was placed at the bottom of the petri dish, the petri dish cover was turned over 180° and placed on the bottom of the petri dish containing sterile water, and vMSC cells were cultured for 24 hours under conditions of 37°C and 5% CO₂ to form spheroids (Hanging drop in FIG. 10). Thereafter, the vMSC spheroids were suspended in M199 medium containing 0.95% methylcellulose, and a Type I collagen solution, which was mixed with 30 mg/mL of type I collagen Gelatin, 10x Medium (Sigma-Aldrich), and 10x reconstitution buffer (0.05 N NaOH, 0.261 M NaHCO₃, and 0.2 M HEPES) in a ratio of 8:1:1, and the suspended spheroids were mixed in a ratio of 1:1. 0.7 mL of the mixture was dispensed into a 24-well plate, and then polymerized for 30 minutes under conditions of 37°C and 5% CO₂. HGF or VEGF as angiogenesis promoting factors were diluted and treated in 100 µl of M199 medium at various concentrations to a gelled gel including the vMSC spheroids, and cultured for 48 hours. Alternatively, instead of the angiogenesis promoting factors, while vMSCs were dispensed and co-cultured with vMSC spheroids in a non-contact manner, 500 ng/mL of HGF-neutralizing antibody (MAB294, R&D systems; Anti-HGF Ab) was mixed and treated into the M199 medium to neutralize HGF, one of the secretomes of vMSCs (sprouting assay in FIG. 10). Sprouting images of the spheroids were obtained by photographing with a microscope (Nikon), and the number of sprouts, cumulative sprouting length (sum of all sprout lengths formed from one spheroid), and average sprouting length (average of all sprout lengths formed from all spheroids) were quantitatively analyzed using Image J software (NIH).

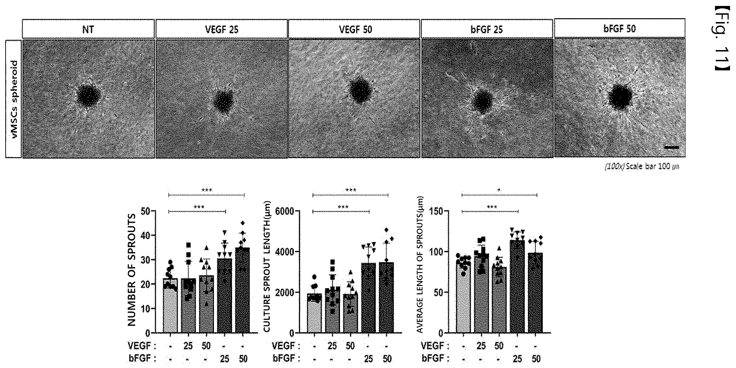

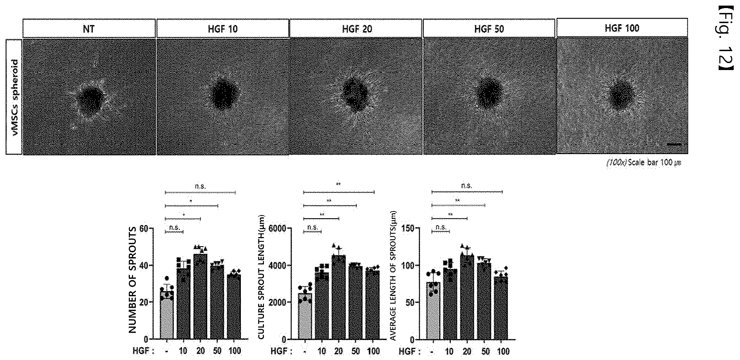

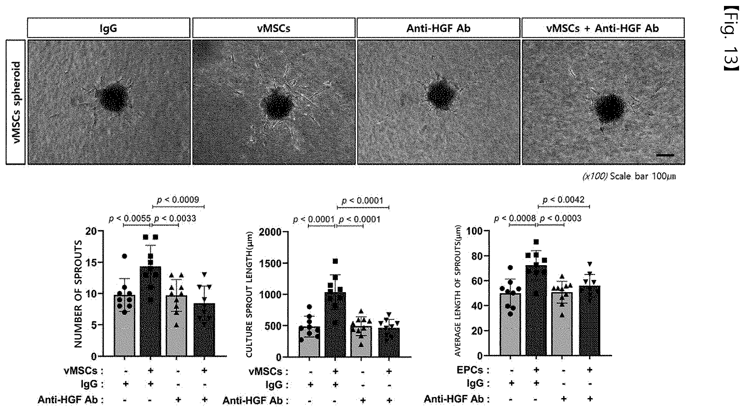

As a result, the vMSC spheroids reacted with bFGF to promote sprouting, but did not react with VEGF (FIG. 11), and sprouting was promoted at concentrations of 20 ng/mL and 50 ng/mL of HGF (FIG. 12). In addition, when the vMSC spheroids were contacted with the vMSC secretomes, which were confirmed to be rich in HGF by non-contact co-culture with vMSCs, the sprouting of vMSC spheroids was promoted, and the increased sprouting was inhibited by HGF-neutralizing antibodies (FIG. 13), and thus it may be suggested that the sprouting ability of vMSC spheroids promoted by the secretomes of vMSCs is due to HGF in the secretome, and it may be seen that HGF is a growth factor that exhibits the autocrine effect of vMSCs.

Accordingly, bone marrow-derived CD141⁺vMSCs secrete an excessive amount of HGF associated with angiogenesis, and the secretomes promote the sprouting of CD141⁺vMSCs. Therefore, the secretomes isolated by culturing CD141⁺vMSCs in a serum-free culture medium may be used as an adjuvant for various stem cell therapy products (CD141⁺vMSCs or EPCs) for vascular regeneration.

### Example 6. Paracrine effect of BM-MSC secretomes

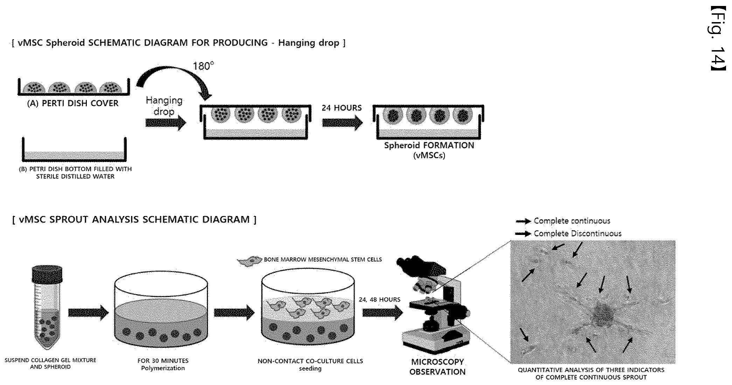

To confirm an effect of vMSCs on promoting vascularization by the secretomes of BM-MSCs, i.e. a paracrine effect, vMSC cells were cultured into spheroids using the hanging drop method as in Example 5 (Hanging drop in FIG. 14), and non-contact co-cultured with BM-MSCs, and then spheroid sprouting assay was performed (sprouting assay in FIG. 14). Specifically, 100 µl of an M199 medium containing 3 × 10⁴ BM-MSCs was dispensed into a gelated gel including vMSC spheroids, and non-contact co-cultured with vMSC spheroids for 48 hours or treated with bFGF (25 ng/mL). At this time, PD173074 (Tocris, Bristol, UK) (10 nM), a bFGF signaling pathway inhibitor, was treated before 3 hours to inhibit bFGF, one of the secretomes of BM-MSCs. Sprouting images of the spheroids were obtained by photographing with a microscope (Nikon), and the number of sprouts, cumulative sprouting length (sum of all sprout lengths formed from one spheroid), and average sprouting length (average of all sprout lengths formed from all spheroids) were quantitatively analyzed using Image J software (NIH). In addition, to compare the mRNA expression levels of bFGF in vMSCs and BM-MSCs, total RNA was isolated from each cell using a TRIzol reagent (Invitrogen), cDNA was synthesized using SuperScript III Reverse Transcriptase (Invitrogen), and then RT-PCR analysis was performed using SYBR Green reagent (Invitrogen). At this time, a human ribosomal protein S9 gene (RPS9) was used as an endogenous control.

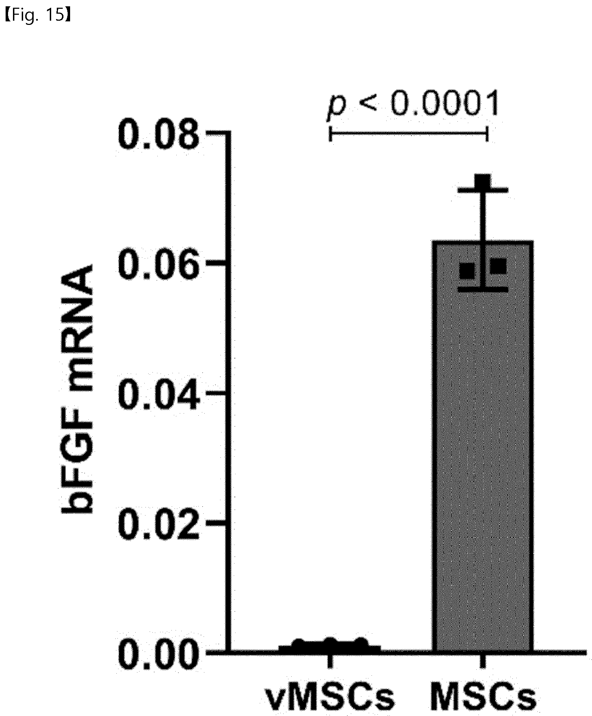

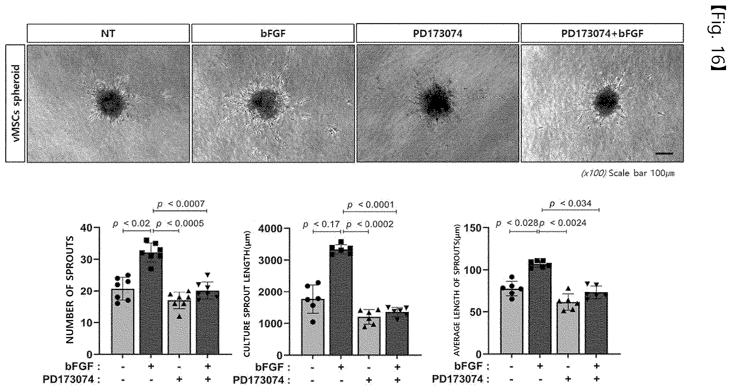

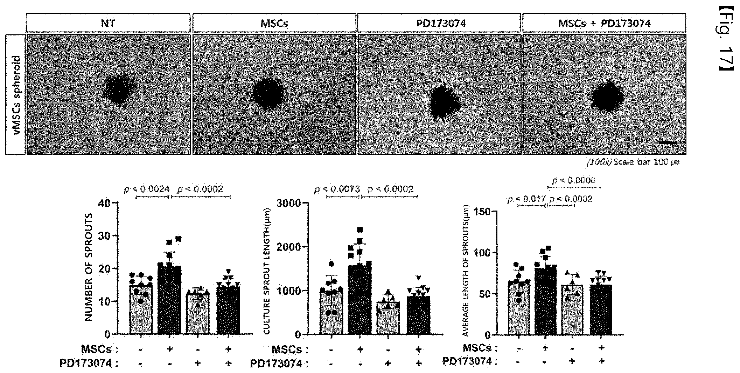

As a result, as the result of comparing the bFGF mRNA levels of vMSCs (vMSCs) and BM-MSCs (MSCs), the expression level of bFGF in BM-MSCs was significantly higher than that in vMSCs (FIG. 15). In addition, the sprouting of vMSC spheroids was promoted by bFGF, which was inhibited by pretreatment with PD173074 for 3 hours (FIG. 16). In addition, when vMSC spheroids and BM-MSCs (MSCs) were non-contact co-cultured, the sprouting of vMSC spheroids was promoted by the secretomes of BM-MSCs, and it was confirmed that the sprouting of vMSC spheroids was inhibited as the result of inhibiting bFGF among the secretomes by pretreating an inhibitor of bFGF (FIG. 17).

Through this, it was found that the secretomes of BM-MSCs (bone marrow-derived mesenchymal stem cells) promoted the sprouting of CD141⁺vMSC spheroids in non-contact co-culture, and the sprouting promoting effect was inhibited by inhibition of the bFGF signaling pathway, so that bFGF secreted by BM-MSCs acted on the angiogenic effect of vMSCs. Therefore, it was confirmed that the sprouting of the vMSC spheroids was promoted by bFGF, not VEGF, and that the secretomes of BM-MSCs that secreted bFGF provided a paracrine angiogenic effect to vMSCs.

Through Examples above, the secretomes of BM-MSCs and the secretomes of vMSCs may be used alone or in combination as additives for pharmaceuticals for promoting vascular regeneration and various stem cell therapy products for vascular regeneration (CD141⁺vMSC or EPC).

## Claims

1. A secretome secreted by stem cells expressing a CD141 (thrombomodulin TM) cell surface antigen.

2. The secretome of claim 1, wherein the stem cells expressing the CD141 cell surface antigen are vasculogenic multipotent stem cells (vMSCs) deposited under the accession number KCLRF-BP-00524.

3. The secretome of claim 1, wherein the secretome includes cell-derived proteins, cytokines, growth factors, exosomes, or nucleic acids.

4. The secretome of claim 1, wherein the secretome includes Endoglin, HGF, IGFBP-2, IL-8, Pentraxin 3, TIMP-1 or uPA.

5. The secretome of claim 1, wherein HGF is increased compared to a secretome secreted from bone marrow-derived mesenchymal stem cells (BM-MSCs).

6. The secretome of claim 1, wherein VEGF is decreased compared to the secretome secreted from the BM-MSCs.

7. A pharmaceutical composition for vascular regeneration or vasculogenesis, comprising a culture solution of stem cells expressing a CD141 cell surface antigen as an active ingredient.

8. The pharmaceutical composition for vascular regeneration or vasculogenesis of claim 7, wherein the stem cells expressing the CD141 cell surface antigen are vasculogenic multipotent stem cells deposited under the accession number KCLRF-BP-00524.

9. The pharmaceutical composition for vascular regeneration or vasculogenesis of claim 7, wherein the culture solution is a culture solution obtained by culturing the stem cells expressing the CD141 cell surface antigen in a serum-free medium.

10. The pharmaceutical composition for vascular regeneration or vasculogenesis of claim 7, further comprising:
a culture solution of bone marrow-derived mesenchymal stem cells.

11. A pharmaceutical composition for vascular regeneration or vasculogenesis, comprising the secretome of claim 1.

12. The pharmaceutical composition for vascular regeneration or vasculogenesis of claim 11, wherein the pharmaceutical composition comprises Endoglin, HGF, IGFBP-2, IL-8, Pentraxin 3, TIMP-1 or uPA.

13. The pharmaceutical composition for vascular regeneration or vasculogenesis of claim 11, wherein the pharmaceutical composition comprises cell-derived proteins, cytokines, growth factors, exosomes, or nucleic acids.

14. The pharmaceutical composition for vascular regeneration or vasculogenesis of claim 11, further comprising:
secretomes secreted from bone marrow-derived mesenchymal stem cells.

15. A stem cell treated with the secretome of claim 1 or a culture solution of a stem cell expressing a CD141 cell surface antigen.

16. The stem cell of claim 15, wherein the stem cell includes cardiac progenitor cells (CPCs), endothelial progenitor cells (EPCs), endothelial colony forming cells (ECFCs), vasculogenic progenitor cells (VPCs), mesenchymal stem cells, embryonic stem cells or myoblasts.

17. The stem cell of claim 16, wherein the mesenchymal stem cells are umbilical cord blood-derived mesenchymal stem cells (UCB-MSCs), umbilical cord-derived mesenchymal stem cells (UC-MSCs), adipose-derived mesenchymal stem cells (AD-MSCs) or bone marrow-derived mesenchymal stem cells (BM-MSCs).

18. The stem cell of claim 15, wherein the stem cell is a stem cell expressing a CD141 cell surface antigen.

19. The stem cell of claim 18, wherein the stem cell expressing the CD141 cell surface antigen is derived from bone marrow, fat, blood, pulp or tonsil.

20. The stem cell of claim 15, wherein a proangiogenic capacity is enhanced by treatment of the secretome or the culture solution.

21. The stem cell of claim 15, wherein a culture solution of bone marrow-derived mesenchymal stem cells or a secretome secreted from the bone marrow-derived mesenchymal stem cells is further treated.

22. A cell therapy product composition for vascular regeneration or vasculogenesis, comprising the stem cell of claim 15 as an active ingredient.

23. A pharmaceutical composition for preventing or treating vascular diseases or vascular dysfunction, comprising the secretome of claim 1 or the stem cell of claim 15 as an active ingredient.

24. The pharmaceutical composition for preventing or treating vascular diseases or vascular dysfunction of claim 23, wherein the vascular disease is traumatic vascular injury, peripheral vascular disease, cardiovascular disease, cerebrovascular disease or ischemic disease.

25. A pharmaceutical composition for preventing or treating brain nervous system diseases, comprising the secretome of claim 1 or the stem cell of claim 15 as an effective ingredient.

26. The pharmaceutical composition for preventing or treating brain nervous system diseases of claim 25, wherein the brain nervous system disease is a degenerative brain disease, a mental disorder, or a developmental disorder.

27. The pharmaceutical composition for preventing or treating brain nervous system diseases of claim 26, wherein the degenerative brain disease is cognitive impairment, Alzheimer's disease, dementia with Lewy bodies, frontotemporal dementia, Parkinson's disease, Creutzfeldt-Jakob disease (CJD), Huntington's disease, multiple sclerosis, or Guillain-Barre Syndrome (GBS).

28. The pharmaceutical composition for preventing or treating brain nervous system diseases of claim 26, wherein the mental disorder is bipolar disorder, autism, depression, hyperactivity, attention deficit, post-traumatic stress disorder (PTSD), anxiety disorder, sleep disorder, panic disorder, intellectual disability, memory impairment, drug addiction, schizophrenia, obsessive-compulsive disorder, delusions of grandeur, personality disorder, alcoholism, or manic depression.

29. The pharmaceutical composition for preventing or treating brain nervous system diseases of claim 26, wherein the developmental disorder is epilepsy, cerebral palsy, developmental language disorder, disturbance of sense, learning disorder, attention deficit hyperactivity disorder (ADHD), autism spectrum disorder (ASD), intellectual disability, cognitive impairment, or impulse control disorders.

30. A method for producing vMSCs-derived secretomes, comprising culturing stem cells expressing a CD141 cell surface antigen in a serum-free medium containing a human platelet lysate.

31. The method for producing vMSCs-derived secretomes of claim 30, further comprising:
filtering the cultured culture solution.

32. A use of secretomes secreted from stem cells expressing a CD141 cell surface antigen, for use in vascular regeneration or vasculogenesis.

33. A use for vascular regeneration or vasculogenesis of stem cells treated with secretomes secreted from stem cells expressing a CD141 cell surface antigen or a culture solution of the stem cells expressing the CD141 cell surface antigen.

34. A method for treating vascular diseases or vascular dysfunction, comprising administering, to a subject having vascular diseases or vascular dysfunction, secretomes secreted from stem cells expressing a CD141 cell surface antigen or a culture solution of the stem cells expressing the CD141 cell surface antigen.

35. A method for preventing or treating brain nervous system diseases, comprising administering, to a subject having brain nervous system diseases, secretomes secreted from stem cells expressing a CD141 cell surface antigen or a culture solution of the stem cells expressing the CD141 cell surface antigen.
